# EUROPÄISCHE PATENTSCHRIFT

(11) **EP 2 177 906 B1**
(45) Veröffentlichungstag und Bekanntmachung des Hinweises auf die Patenterteilung: **03.06.2015**
(21) Anmeldenummer: 09173092.9
(22) Anmeldetag: 15.10.2009
(51) Int. Cl.: G01N 33/44

(54) **Säulenpresse zur Qualitätsanalyse**
Column press for quality analysis
Presse à colonnes pour l'analyse de la qualité

(30) Priorität: 16.10.2008 DE 102008042906
(43) Veröffentlichungstag der Anmeldung: 21.04.2010
(73) Patentinhaber: Rhein Chemie Rheinau GmbH, 68219 Mannheim (DE)
(72) Erfinder: Schuchmann, Martin, 68309 Mannheim (DE); Herbel, Hermann, 67376 Harthausen (DE)
(74) Vertreter: Siegers, Britta

(56) Entgegenhaltungen:
- DE-A1- 4 339 984
- DE-B- 1 237 834
- DE-B- 1 238 198
- DE-C- 877 949

## Beschreibung

Die Erfindung betrifft ein Verfahren und eine Einrichtung zur Prüfung der Qualität von polymergebundenen Kautschukadditiven. Die Prüfung der Qualität eines polymergebundenen Kautschukadditivs erfolgt mittelbar durch Überprüfen einer hergestellten Prüfmischung, die das polymergebundene Kautschukadditiv enthält. Derartige polymergebundene Kautschukadditive sind beispielsweise unter der Bezeichnung Rhenogran® kommerziell erhältlich. Rhenogran® umfasst wenigstens ein Kautschukadditiv und wenigstens einen polymeren Binder. Als Kautschukadditive kommen beispielsweise Schwefel, Vulkanisationsbeschleuniger wie MBTS, Alterungsschutzmittel wie MBI oder auch Aktivatoren wie Zinkoxid in Frage.

Unter dem Begriff Rhenogran® werden beispielsweise Vernetzungsmittel angeboten, um mit Hilfe des Vernetzu>ngsmittels aus einem Kautschuk ein Elastomer herzustellen. "Rhenogran® S-80" ist ein solches Vernetzungsmittel, welches zu 80 % aus Schwefel und 20 % aus Polymerbindern besteht.

Ein weiteres Beispiel aus der Rhenogran®-Produktpalette heißt Renogran® MBI-80 und basiert auf dem Alterungsschutzmittel MBI. Renogran MBI-80 weist 80 % Wirkstoff MBI und 20 % Binder auf. Beim Wirkstoff MBI handelt es sich um Mercaptobenzimidazol.

Rhenogran® - Additive werden beispielweise für die Herstellung von aus Gummi bestehenden Produkten eingesetzt.

In Abhängigkeit von der Qualität eines polymergebundenen Kautschukadditivs können im vulkanisierten Endprodukt Stippen auftreten, also optisch sichtbare Veränderungen. Damit können technische Mängel verbunden sein. Je weniger Stippen im Endprodukt auftreten, umso höher ist die Qualität des Polymer-gebundenen Kautschukadditivs.

Aus der Druckschrift DD 159718 ist ein Verfahren zur Herstellung von Verschnitten aus Butadien-Acrylnitril-Kautschuken und Polyvinylchlorid bekannt, die durch Vermischen von Butadien-Acrylnitril-Latices und Polyvinylchlorid-Latices entstehen. Im festen Endprodukt können sich laut dieser Druckschrift durch mitgetragene Ausscheidungsteilchen Stippen bilden, die eine Verminderung der Produkt-Qualität hervorrufen und zu Schwierigkeiten bei der Weiterverarbeitung führen. Dass Stippen die Produktqualität vermindern, ist ferner aus der Druckschrift DE 10 2004 048 098 A1 bekannt.

Aus der Druckschrift DE 10 2005 019 395 A1 geht hervor, die Mischgüte eines direkt aus einem Reaktor erhaltenen Polyethylenpulvers zu prüfen, indem dünne Scheiben (Mikrotomschnitte) einer Probe unter dem Lichtmikroskop begutachtet werden. Je besser die Mischgüte eines Polymers ist, um so weniger und um so kleinere, als Stippen bezeichnete Einschlüsse werden festgestellt. Quantitativ wird die Mischgüte eines Polymers nach ISO 13949 bestimmt. Nach der Messvorschrift wird ein Mikrotomschnitt aus einer Probe des Polymers angefertigt, Anzahl und Größe der Einschlüsse werden ausgezählt, und nach einem festgelegten Bewertungsschema wird eine Note für die Mischgüte des Polymers festgelegt.

Es ist Aufgabe der Erfindung, ein Verfahren bereit zu stellen, mit der zuverlässig die Qualität eines polymergebundenen Kautschukadditivs geprüft werden kann sowie eine Einrichtung zur Durchführung des Verfahrens.

Zur Lösung der Aufgabe wird eine Prüfmischung hergestellt, die das zu prüfende polymergebundene Kautschukadditiv und ein Prüfbatch umfasst. Das Prüfbatch enthält vorzugsweise einen oder mehrere Kautschuke, Ruß, helle Füllstoffe, Weichmacheröl, Vernetzungsaktivator und/ oder Verarbeitungswirkstoffe. Zur Herstellung der Prüfmischung wird insbesondere polymergebundenes Kautschukadditiv - beispielsweise 100g - und schwarzes EPDM Batch - beispielsweise 300 g - verwendet. Schwarzes EPDM Batch enthält nicht nur das Polymer EPDM, also Ethylen-Propylen-Dien-Kautschuk, sondern darüber hinaus weitere Zusätze, und zwar insbesondere erhebliche Mengen an Ruß sowie Öle als Weichmacher. Mit der Verwendung eines Prüfbatch wird angestrebt, bereits möglichst gut ein tatsächlich verwendetes Material nachzuahmen, um so zuverlässig die Qualität prüfen zu können. Dieses Ziel wird beispielsweise durch Vorsehen eines EPDM Batches als Prüfbatch erreicht. Anstelle von EPDM können Kautschuke wie Naturkautschuk (NR) oder andere Synthesekautschuke wie z. B. Styrolbutadienkautschuk (SBR), Butadienkautschuk (BR), Polyisoprenkautschuk (IR), Butylkautschuk (IIR), Nitrilbutadienkautschuk (NBR) und hydrierter NBR (HNBR), Polychloroprenkautschuk (CR), Ethylenpropylenkautschuk (EPM), Acrylatkautschuke (AEM, ACM), Fluorkautschuk (FKM), Epichlorohydrinkautschuk (ECO), Ethylenvinylacetatkautschuk (EVM) oder Mischungen der vorgenannten Kautschuke verwendet werden.

Zunächst wird das polymergebundene Kautschukadditiv in ein Prüfbatch eingemischt, insbesondere in schwarzes EPDM Batch und zwar für jede Qualitätsprüfung in gleicher Weise. Auf einer temperierten Walze mit definiertem Walzenspalt wird das polymergebundene Kautschukadditiv bevorzugt in das Prüfbatch wie das schwarze EPDM Batch eingemischt. Die Walze wird insbesondere temperiert, um verarbeitungsgerechte Viskositäten zu erhalten. Temperaturen zwischen 50 und 80 °C sind für die Temperierung regelmäßig geeignet und zwar insbesondere im Fall des schwarzen EPDM Batch.

Es gibt vier Parameter, die ein mittels temperierter Walze erzieltes Mischergebnis beeinflussen. Ein Parameter ist die Temperatur, mit der eine Walze temperiert wird. Ein zweiter Parameter ist der Walzenspalt und ein dritter die Mischdauer. Die Handhabung, wie polymergebundenes Kautschukadditiv und Prüfbatch zugeführt werden, ist ein vierter Parameter. Diese vier Parameter werden für die Durchführung von Qualitätskontrollen nicht verändert, um zu vergleichbaren Ergebnisse zu gelangen, die unmittelbar eine Aussage über die Qualität der jeweils untersuchten Prüfmischung erlauben. Daher erfolgt u. a. die Zuführung in stets gleicher Weise, um nicht aufgrund einer nicht vergleichbaren Handhabung unterschiedliche Ergebnisse zu erhalten.

Die vorgegebene Dicke des Walzenspaltes beeinflusst maßgeblich die Scherenergie, die in die Mischung eingetragen wird. Der Walzenspalt wird so gewählt, dass ein gewünschtes Dispersionsergebnis erhalten wird. Der einmal so ausgewählte Walzenspalt wird auch für Folgeuntersuchungen beibehalten.

Nach Ablauf der vorgegebenen Mischdauer wird das Gemisch durch ein Sieb gepresst (nachfolgend auch Prüfsieb genannt), so zum Beispiel mit Hilfe einer Schnecke. Ein dafür geeigneter Extruder mit Sieb und Schnecke wird beispielsweise von der Firma GÖTTFERT Werkstoff-Prüfmaschinen GmbH, Buchen, Deutschland, angeboten. Der Extruder umfasst eine Einzugsschnecke, mit der die Prüfmischung zu einem Sieb transportiert und durch das Sieb gepresst wird. Nachdem die Prüfmischung vollständig extrudiert worden ist, wird das Sieb, auch unter der Bezeichnung Strainervorsatz bekannt, demontiert. Der im Strainervorsatz zurück gebliebene Siebkuchen wird entnommen, also vom Sieb gelöst und unter dem Mikroskop betrachtet. Es werden so Stippen anhand von Größe und Anzahl ausgezählt. Je mehr und je größere Stippen gefunden werden, umso schlechter ist das Ergebnis. Die Bewertung erfolgt insbesondere gemäß standardisierten Bewertungsverfahren. Kategorien werden im Rahmen eines solchen standardisierten Bewertungsverfahrens vorgegeben und zwar insbesondere unterteilt nach Größe der Stippen. So kann eine erste Kategorie sich auf 75 µm bis 175 µm große Stippen beziehen, eine zweite Kategorie auf 175 µm bis 250 µm große Stippen und eine dritte Kategorie auf Stippen größer als 250 µm. Pro Kategorie wird zum Beispiel nur eine bestimmte Anzahl an Stippen zugelassen. Wird diese vorgegebene Anzahl überschritten, entspricht das polymergebundene Kautschukadditiv beispielsweise nicht den Qualitätsanforderungen und gelangt nicht in den ursprünglich vorgesehenen Verkauf.

Für derartige Transportaufgaben wird nach dem Stand der Technik üblicherweise eine Schnecke eingesetzt, da eine Schnecke aus nachfolgend genannten Gründen für eine solche Aufgabe besonders gut geeignet ist.

Um die Prüfmischung der Aufnahmezone einer Schnecke zuführen zu können, wird die Prüfmischung zunächst geeignet dimensioniert, so zum Beispiel in Streifen geschnitten. Kühlt die Prüfmischung ab, so wird die Viskosität regelmäßig derart hoch, dass die Prüfmischung nicht mehr transportiert und durch ein Sieb gepresst werden kann, obwohl gerade mit einer Schnecke sehr große Kräfte erzeugt werden können. Daher wird vor allem auch die Schnecke temperiert, um großflächig Wärme auf die Prüfmischung zu übertragen. Es gelingt so insbesondere, dass die mit der Schnecke transportierte Prüfmischung umfassend erwärmt wird. Denn aufgrund der während des Transports auftretenden Gegenkräfte strömt ein Teil der Prüfmischung zurück, so dass sich die Prüfmischung innerhalb des Extruders weiter durchmischt und die für die gewünschte Viskosität benötigte Wärme schnell verteilt wird. Auch entsteht dabei Reibungswärme, die weiter dazu beiträgt, dass die Viskosität der Prüfmischung nicht zu sehr ansteigt.

Mit einer Schnecke kann selbst dann unproblematisch transportiert werden, wenn Viskositätsschwankungen verkraftet werden müssen. Steigt die Viskosität aufgrund einer Schwankung stellenweise an, so kann dies im Fall einer Schnecke durch das Zurückfließen von Material kompensiert werden. Eine Schnecke bleibt aufgrund von Viskositätsschwankungen nicht stehen und wird auch nicht beschädigt.

Der Rückfluss von Material innerhalb einer Vorrichtung, bei der der Transport durch eine Schnecke bewirkt wird, ist zwar mit einigen wichtigen Vorteilen verbunden, um das Verfahren ausführen zu können. Allerdings wird das Gemisch durch den Rückfluss in nicht vorhersehbarer Weise gemischt. Dieses Mischen aufgrund des Transports mit einer Schnecke verändert nachteilhaft das gesuchte Resultat in nicht reproduzierbarer Weise.

In einer besonders bevorzugten Ausführungsform der Erfindung wird daher das Gemisch in einem Zylinder mit einem Kolben durch ein Sieb gepresst, welches an einem Ende des Zylinders befestigt ist.

Zwar ist ein Transport des Gemisches mit Kolben und Zylinder aus mehreren Gründen problematisch. So ist es nicht möglich, die Prüfmischung geeignet zu temperieren, um so die Viskosität nicht derart ansteigen zu lassen, dass ein Transport nicht mehr möglich ist. Denn bei einem Zylinder nebst Kolben kann Wärme nur von außen zur Prüfmischung geleitet werden. Es gelingt bereits deshalb nicht, Wärme schnell genug gleichmäßig in die Prüfmischung hinein zu transportieren. Da die Prüfmischung im Zylinder nur noch transportiert, aber nicht mehr aufgrund eines Rückflusses zusätzlich gemischt wird, fehlt ein damit einhergehender Wärmeaustausch, der im Fall des Extruders die gewünschte Temperierung der Prüfmischung unterstützt.

Viskositätsschwankungen können nicht mehr durch einen verstärkten Materialrückfluss kompensiert werden. Es droht daher im Fall eines Transports mittels Kolben und Zylinder ein Stillstand, eine Beschädigung des Antriebs oder des Siebs.

Allerdings ist mit dem Transport in einem Zylinder ein wesentlicher Vorteil verbunden. Da nämlich die Prüfmischung nicht mehr während des Transports zusätzlich gemischt wird, wird das Ergebnis der Qualitätskontrolle nicht durch ein Mischen aufgrund des Transports verändert.

Es wurde überraschend festgestellt, dass die mit einem Transport mittels Kolben und Zylinder verbundenen Probleme gelöst werden können. So steht im Fall eines Zylinders die gesamte Grundfläche zur Verfügung, um eine Prüfmischung in den Zylinder einfüllen zu können. Die Prüfmischung muss daher nicht mehr in der aus dem Stand der Technik bekannten Weise portioniert, so zum Beispiel in Streifen geschnitten werden, um eine Transportvorrichtung (also beispielsweise einen Extruder) mit der Prüfmischung zu füllen. Die zu untersuchende Prüfmischung wird geeignet zylinderartig geformt und in den Zylinder hineingeschoben. Da dies sehr schnell geschehen kann, kühlt die Prüfmischung kaum aus, so dass die Viskosität zu diesem Zeitpunkt nicht zu stark ansteigt. Es hat sich herausgestellt, dass nun die so in einen Zylinder eingefüllte Prüfmischung sehr viel schneller durch das Prüfsieb gepresst werden kann. Denn einerseits wird während des Transports nicht mehr gemischt. Im Unterschied zum Extruder führt eine erhöhte Transportgeschwindigkeit auch nicht verstärkt zu einem Mischen während des Transports, was verstärkt das Ergebnis der Qualitätskontrolle verfälschen würde, wodurch die Transportgeschwindigkeit nach oben hin limitiert wird. Auch hat sich herausgestellt, dass die Viskosität einer Prüfmischung aufgrund der möglichen Verarbeitungsgeschwindigkeiten während des Transports nicht derart ansteigt, dass hierdurch ein relativ schneller Transport verhindert wird. Es hat sich herausgestellt, dass dann außerdem die Viskositätsschwankungen nicht so ausgeprägt sind, dass dadurch der Transport mit der gewünschten bzw. erforderlichen Geschwindigkeit unterbunden wird.

In einer Ausführungsform der Erfindung wird wie bereits beschrieben, mit Hilfe einer Doppelwalze polymergebundenes Kautschukadditiv mit dem Prüfbatch gemischt. Die so hergestellte Prüfmischung kann schließlich in Form einer Matte (Mischungsfell) temperiert erhalten . Die Matte (Mischungsfell) kann sofort so gerollt werden, dass die gewünschte Zylinderform entsteht. Auf einfache Weise kann die gewünschte Zylinderform mit der erforderlichen Geschwindigkeit erhalten werden, so dass die durch die Walze temperierte Prüfmischung nicht zu stark abkühlt. Alternativ dazu kann aber auch auf andere Weise gemischt und dann mit Hilfe einer Walze zunächst die Prüfmischung in die Form einer Matte gebracht und aufgerollt werden. Allerding erfordert diese Variante einen zusätzlichen Arbeitsschritt. Mit diesen beiden Ausführungsformen lassen sich jedenfalls besonders zuverlässig die Probleme überwinden, die mit dem Transport mittels Zylinder und Kolben verbunden sind.

Wird ein Zylinder nebst Kolben für den Transport eingesetzt, so kann damit außerdem der Reinigungsaufwand reduziert werden, da sich ein Zylinder nebst Kolben im Vergleich zu einer Schnecke nicht nur leichter reinigen lässt, sondern auch weniger verunreinigt wird, da der Kolben Ablagerungen auf Innenwänden des Zylinders abstreift.

Der Zylinder steht in der Regel senkrecht. Das dann oberste Sieb hat eine typische Maschenweite von 150µm, alle darunter liegen Siebe dienen als Stützsiebe und haben eine größere Maschenweite. Ein typischer Aufbau umfasst eine Lochplatte, die als Klemmschutz dient. Im Sieb mit der Maschenweite von 150µm (dem Sieb mit der kleinsten Maschenweite) bleiben dann alle Stippen größer 150µm "hängen".

Nachfolgend kann ein Stützsieb mit einer Maschenweite von 250µm vorgesehen sein, welches als Unterlage für das 150 µm Sieb dient. Hieran kann sich ein Stützsieb mit einer Maschenweite von 500 µm anschließen, das als Unterlage für das 250 µm Sieb dient. Einen Abschluss kann eine Lochscheibe bilden, auf der alle Siebe liegen.

Die Erfindung umfasst ein Verfahren zur Prüfung von polymergebundenen Kautschukadditiven, gemäß dem ein polymergebundenes Kautschukadditiv mit einem Prüfbatch, insbesondere einem EPDM Batch gemischt wird, die Prüfmischung mit wenigstens einer Walze (1, 1a) zu einer Matte geformt wird, die Matte aufgerollt und in aufgerollter Form (5) durch ein Prüfsieb gepresst wird und die Stippen ermittelt werden, die ausgesiebt wurden.

In einer Ausgestaltung des Verfahrens wird die Prüfmischung mit Hilfe eines Kolbens (7) und eines Zylinders (6) durch das Prüfsieb (10) gepresst.

In einer weiteren Ausgestaltung des Verfahrens wird eine erwärmte Prüfmischung (5) zunächst in die Form einer Rolle oder eines Zylinders gebracht wird, die so geformte Prüfmischung (5) in einen Zylinder (6) gesteckt und mit Hilfe eines Kolbens (7) durch das Prüfsieb (10) gepresst.

Die Erfindung umfasst außerdem eine Ausgestaltung des Verfahrens, bei dem polymergebundenes Kautschukadditiv (3) und Prüfbatch (2)mittels einer Doppelwalze (1, 1a) gemischt werden.

Die Erfindung betrifft ferner eine Ausgestaltung des Verfahrens, bei dem eine Mehrzahl von Sieben und/ oder siebförmigen Platten (8, 9, 10) in Pressrichtung gesehen hinter dem Prüfsieb (10) aneinandergrenzend angeordnet sind, wobei die Maschengröße der einzelnen Siebe (8, 9, 10) in Pressrichtung zunimmt.

Die Erfindung umfasst eine Ausgestaltung des Verfahrens, bei dem vor dem Prüfsieb (10) ein Ring (11) angeordnet ist.

Die Erfindung betrifft außerdem eine Einrichtung eingerichtet zur Durchführung des Verfahrens mit einem Zylinder (6) und einem darin geführten Kolben (7), mit einer Mehrzahl von Sieben (8,9, 10) an einem Ende des Zylinders und einem Ring angrenzend an ein Sieb (10).

Die Erfindung umfasst eine Ausgestaltung der Einrichtung, wobei ein Sieb (10) eine Maschenweite von nicht mehr als 150 µm aufweist und die Maschen der sich daran nach außen anschließenden Siebe (8, 9) größer sind.

Die Erfindung betrifft eine Ausgestaltung der Einrichtung umfassend eine Doppelwalze (1a, 1b) nebst Begrenzungsplatten.

Die möglichen für die Prüfung eingesetzten Mengen sind durch die Größe der Aggregate festgelegt. Die maximale Menge ist durch die Größe des Zylinders terminiert.

Nachfolgend wird die Erfindung anhand eines Beispiels näher erläutert, ohne dabei auf dieses beschränkt zu sein.

### Ausführungsbeispiel

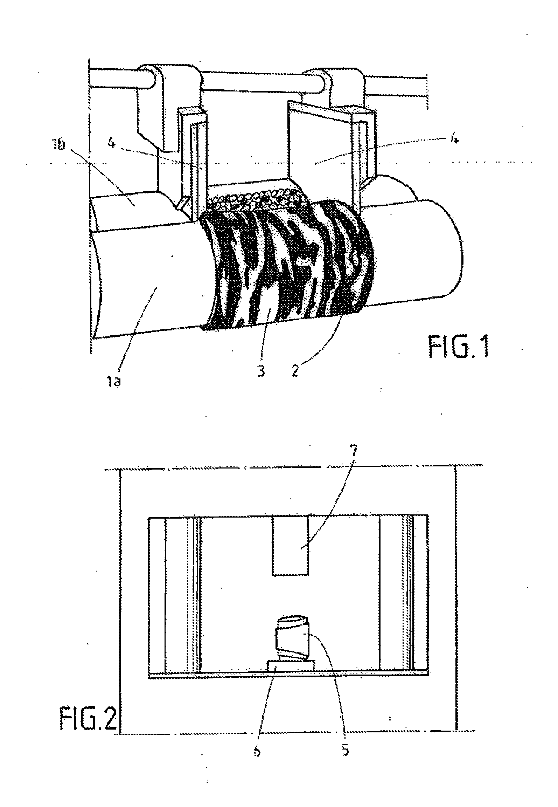

Figur 1 zeigt eine kommerzielle, temperierte Doppelwalze, bestehend aus zwei hintereinander angeordneten Stahlzylindern 1a und 1 b. Auf den Stahlzylindern befindet sich zum einen EPDM Batch 2 und zum anderen ein Produkt der Rhenogran® -Produktpalette 3. Die Spaltbreite zwischen den beiden Walzen liegt in der Regel im Millimeterbereich, beispielsweise zwischen 1 und 10 mm, vorzugsweise bis 3 mm. Ist für die Durchführung einer Qualitätskontrolle eine Spaltbreite gewählt worden, so wird diese auch bei folgenden Qualitätskontrollen beibehalten.

Das verwendete EPDM Batch (Batch-Nr. 9-318 EPDM) war wie folgt zusammengesetzt:

| | |
|---|---|
| Buna^{®} EPG 5450 | 100 phr |
| Ruß N 550 | 90 phr |
| Sillitin N 85 | =50 phr, Kieselsäure mit Kaolinit, erhältlich bei der Firma Hoffman Mineral GmbH |
| Paraffinöl | 75 phr |
| ZnO Aktiv | 5 phr |
| PEG 4000 | 2 phr = Polyethylenglykol 4000 erhältlich bei der Firma Brühl - Chemikalien Handel GmbH |
| Stearinsäure | 1 phr |
| Rhenosorb C/ Rhenofit C | 5 phr = Calciumoxid, erhältlich bei der Firma Rhein Chemie Rheinau GmbH |
| | 328 phr |

Bei Buna^{®} handelt es sich um einen Ethylen-Propylen-Dien-Kautschuk, erhältlich bei der Firma LANXESS Deutschland GmbH mit einem EthylenGehalt von 52 ± 4 Gew.% und einer Mooney-Viskosität ML von (1+4) 46 ± 5 MU bei 125°C.

Der dem Fachmann geläufige Ausdruck phr ("parts per hundred rubber") gibt die Gewichtsteile in der Zusammensetzung an.

300 g des EPDM-Batches werden abgewogen und auf die Walze gegeben. 100 g des jeweiligen Rhenogran-Produkts (z. B. Rhenogran TBzTD-70) = Tetrabenzylthiuramdisulfid, erhältlich bei der Firma Rhein Chemie Rheinau GmbH werden in Form von Granulat zugeführt. Das Granulat wird eingestreut, sobald der Batch warm genug geworden und die Viskosität genügend niedrig ist.

Oberhalb der Walzen 1a, 1b an diese angrenzend befinden sich zwei Begrenzerplatten 4, die dafür sorgen, dass sich das Material 2, 3 nicht unkontrolliert ausbreitet.

Das Mischen wird manuell wie folgt durchgeführt. Mit einem Messer schneidet eine Person beispielsweise ein Stück schräg heraus und legt es an anderer Stelle auf die vordere Walze 1a wieder auf.

Ist das gesamte Material 2, 3 eingearbeitet und dispergiert worden, so ist die so hergestellte Prüfmischung homogen schwarz gefärbt. Die Prüfmischung wird parallel zur Achse der Walzen aufgeschnitten, abgezogen und zylinderförmig aufgerollt. Es ist aber auch möglich, die abgezogene und aufgerollte Prüfmischung bzw. das aufgerollte Mischungsfell in einem Zwischenschritt noch ein oder mehrmals durch das Walzenpaar zu führen und dadurch weiter zu homogenisieren. Die Prüfmischung liegt abschließend in Form einer Matte (Mischungsfell) vor, die aufgerollt wird. Die Temperatur dieser Rolle liegt dann typischerweise bei ca. 60°C.

Die so erhaltene, regelmäßig ca. 60°C warme Rolle 5 wird in einen dafür vorgesehenen Zylinder 6 hineingesteckt, wie Figur 2 verdeutlicht. Mit einem in Figur 2 gezeigten Kolben 7 wird die rollenförmige Prüfmischung 5 durch ein Sieb hindurchgedrückt. Der Durchmesser des gezeigten Zylinders beträgt beispielsweise 3 bis 10 cm. Die Länge des Zylinders beträgt beispielsweise 20 bis 60 cm.

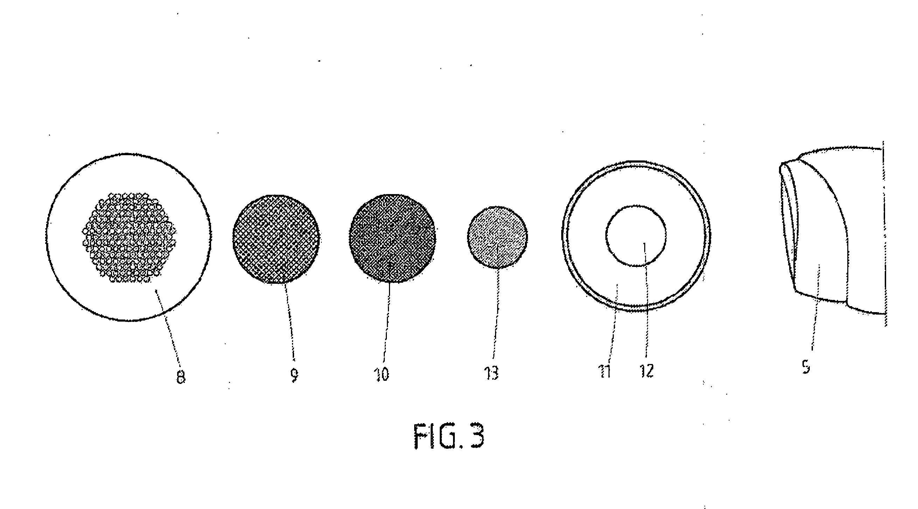

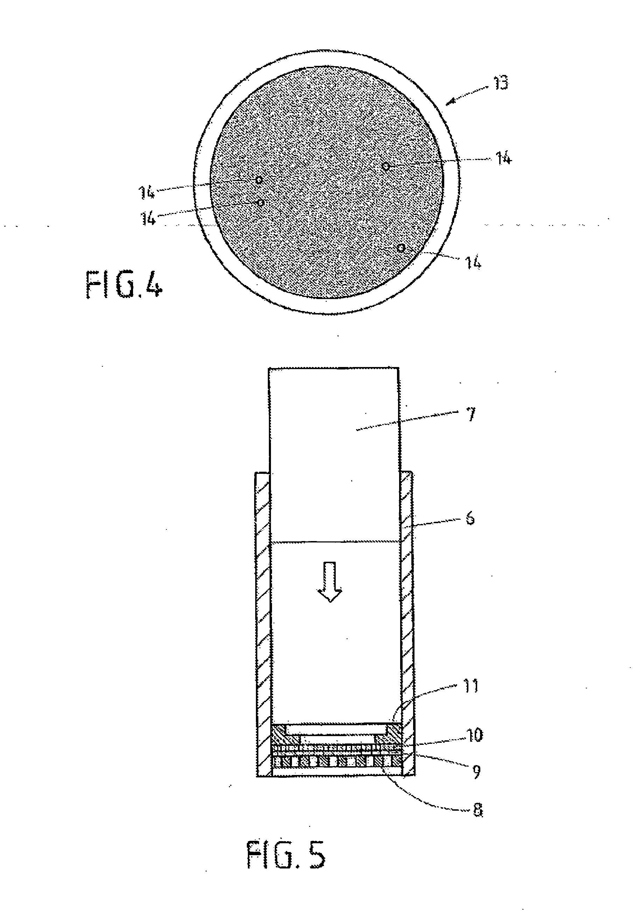

Figuren 3 und 5 verdeutlichen den Aufbau des unteren Endes des Zylinders, das ein Sieb umfasst. Figur 5 zeigt den Aufbau im Schnitt.

Das untere Ende ist zunächst mit einer aus Stahl bestehenden siebförmigen, relativ dicken Platte 8 versehen, die als erste Stütze dient, um den auftretenden Drücken gewachsen zu sein. Die Platte 8 ist mit einer Vielzahl von relativ großen Löchern versehen. Oberhalb der siebförmigen Platte 8 wird ein vergleichsweise dünnes Sieb 9 aufgelegt, welches eine Maschenweite von ca. 500 µm aufweisen kann und ebenfalls als Stütze dient. Auf dem Stützsieb 9 liegt dann das ebenfalls relativ dünne Prüfsieb 10 mit einer Maschenweite von ca. 150 µm auf. Die Maschenweite des Prüfsiebs 10 wird regelmäßig so gewählt, dass die Maschen die Stippen hindurch lassen, die für zulässig erachtet werden. Stützsieb und siebförmige Platten weisen zunehmend größer Maschen auf. Ausgesiebt werden dann nur solche Stippen, die als zu groß erachtet werden.

Das Prüfsieb 10 ist einem sehr hohen Druck während des Transports ausgesetzt, der 10 bis 100 bar betragen kann. Diesem Druck kann das Prüfsieb 10 aufgrund des nachgelagerten Stützsiebs 9 und der siebförmigen Platte 8 standhalten.

Vor dem Prüfsieb 10 wird ein Metallring 1 1 angeordnet. Wird die Prüfmischung - soweit möglich - praktisch vollständig durch das Prüfsieb 10 hindurch gedrückt, so verbleiben die in der Prüfmischung vorhandenen Stippen schließlich in dem Bereich der Ausnehmung 12 des Metallrings 11. Der in der Ausnehmung verbliebene Teil 13 der Probe, also der Siebkuchen kann leicht entnommen und geprüft werden. Der Siebkuchen 13 weist dann bereits eine geeignete Größe und Dicke auf, um diesen unter einem Mikroskop betrachten und analysieren zu können.

Figur 4 verdeutlicht ein Ergebnis, welches für Rhenogran TBzTD-70 erhalten wurde. Es wurden in dem gezeigten Fall eine Stippe der Kategorie 1 mit einem Durchmesser von 150 - 175 µm gefunden, drei Stippen der Kategorie 2 mit einem Durchmesser von 175 - 250 µm und vier in der Figur 4 noch erkennbare Stippen 14 der Kategorie 3 mit einem Durchmesser von mehr als 250 µm.

Die Prüfmischung und damit auch das in der Mischung befindliche polymergebundene Kautschukadditiv waren damit mangelhaft.
Die beim Ausführungsbeispiel eingesetzte Prüfmengen betrugen minimal insgesamt 200 g und maximal insgesamt 640 g. Wenn andere Anlagen mit anderen Geometrien zum Einsatz kommen, dann sind auch andere Mengen möglich, so zum Beispiel bei Einsatz eines größeren Zylinders.

Erfindungsgemäß wurde ein Mengenverhältnis von 100 g zu prüfendes Rhenogran und 300 g schwarzes EPDM Batch eingesetzt. Das Mengenverhältnis betrug also 1 :3. Es sind aber auch andere Mengenverhältnisse möglich, so zum Beispiel von 1:2 oder 1:4. Bei zu großer Konzentration an Rhenogran^{®} können Fehler aufgrund zu vieler Stippen auftreten, die für den Operator nicht mehr auswertbar sind, da sich ein Belag an Stippen gebildet hat und somit ein Auszählen unmöglich macht. Bei zu kleiner Konzentration an Rhenogran können zu wenig Stippen auftreten. Das Ergebnis bei zu wenig Stippen ist dann gegebenenfalls statistisch nicht mehr relevant.

## Patentansprüche

1. Verfahren zur Prüfung von polymergebundenen Kautschukadditiven (3), **dadurch gekennzeichnet, dass** ein polymergebundenes Kautschukadditiv (3) mit einem Prüfbatch, insbesondere einem EPDM Batch (2) gemischt wird, die Prüfmischung mit wenigstens einer Walze (1a, 1b) zu einer Matte geformt wird, die Matte aufgerollt und in aufgerollter Form (5) durch ein Prüfsieb (10) gepresst wird und die Stippen (14) ermittelt werden, die ausgesiebt wurden.

2. Verfahren nach Anspruch 1, bei dem die Prüfmischung (5) mit Hilfe eines Kolbens (7) und eines Zylinders (6) durch das Prüfsieb (10) gepresst wird.

3. Verfahren nach Anspruch 1 oder 2, bei dem eine erwärmte Prüfmischung (5) zunächst in die Form einer Rolle oder eines Zylinders gebracht wird, die so geformte Prüfmischung (5) in einen Zylinder (6) gesteckt wird und mit Hilfe eines Kolbens (7) durch das Prüfsieb (10) gepresst wird.

4. Verfahren nach einem der vorhergehenden Ansprüche, bei dem polymergebundenes Kautschukadditiv (3) und Prüfbatch (2) mittels einer Doppelwalze (1a, 1b) gemischt werden.

5. Verfahren nach einem der vorhergehenden Ansprüche, bei dem eine Mehrzahl von Sieben und/ oder siebförmigen Platten (8, 9, 10) in Pressrichtung gesehen hinter dem Prüfsieb (10) aneinandergrenzend angeordnet sind, wobei die Maschengröße der einzelnen Siebe (8, 9, 10) in Pressrichtung zunimmt.

6. Verfahren nach einem der vorhergehenden Ansprüche, bei dem vor dem Prüfsieb (10) ein Ring (11) angeordnet ist.

7. Einrichtung eingerichtet zur Durchführung des Verfahrens nach einem der vorhergehenden Ansprüche mit einem Zylinder (6) und einem darin geführten Kolben (7), mit einer Mehrzahl von Sieben (8, 9, 10) an einem Ende des Zylinders und einem Ring (11) angrenzend an ein Sieb (10).

8. Einrichtung nach dem vorhergehenden Anspruch, wobei ein Sieb (10) eine Maschenweite von nicht mehr als 150 µm aufweist und die Maschen der sich daran nach außen anschließenden Siebe (8, 9) größer sind.

9. Einrichtung nach einem der beiden vorhergehenden Ansprüche umfassend eine Doppelwalze (1a, 1b) nebst Begrenzungsplatten (4).

## Claims

1. Method for the testing of polymer-bound rubber additives (3), **characterized in that** a polymer-bound rubber additive (3) is mixed with a test masterbatch, in particular with an EPDM masterbatch (2), the test mixture is moulded using at least one roller (1a, 1b) to give a mat, and the mat is rolled up and, in rolled-up form (5), forced through a test sieve (10), and the number of undispersed particles (14) removed by sieving is determined.

2. Method according to Claim 1, in which the test mixture (5) is forced through the test sieve (10) with the aid of a piston (7) and of a cylinder (6).

3. Method according to Claim 1 or 2, in which a heated test mixture (5) is first converted to the form of a roll or of a cylinder, and the test mixture (5) thus moulded is inserted into a cylinder (6), and is forced through the test sieve (10) with the aid of a piston (7).

4. Method according to any of the preceding claims, in which polymer-bound rubber additive (3) and test masterbatch (2) are mixed by means of a double-roll system (1a, 1b).

5. Method according to any of the preceding claims, in which a plurality of sieves and/or of plates (8, 9, 10) in the form of a sieve have been arranged adjacent to one another behind the test sieve (10), seen in the direction of pressure, and the mesh size of each of the sieves (8, 9, 10) here increases in the direction of pressure.

6. Method according to any of the preceding claims, in which a ring (11) has been arranged in front of the test sieve (10).

7. Equipment set up for carrying out the method according to any of the preceding claims with a cylinder (6) and with a piston (7) conducted therein, with a plurality of sieves (8, 9, 10) at one end of the cylinder and with a ring (11) adjacent to a sieve (10).

8. Equipment according to the preceding claim, where a sieve (10) has a mesh width of not more than 150 µm and the meshes of the sieves (8, 9) adjacent thereto towards the outside are greater.

9. Equipment according to either of the two preceding claims encompassing a double-roll system (1a, 1b) together with delimiter plates (4).

## Revendications

1. Procédé pour le contrôle d'additifs pour caoutchouc (3) liés à des polymères, **caractérisé en ce qu'**on mélange un additif pour caoutchouc (3) lié à un polymère avec une charge d'essai, en particulier une charge d'EPDM (2), on met le mélange d'essai en forme d'un mat à l'aide d'au moins un cylindre (1a, 1b), on enroule le mat et sous forme enroulée (5) on le presse à travers un tamis d'essai (10) et on détermine les grains (14) qui ont été extraits par tamisage.

2. Procédé selon la revendication 1, dans lequel le mélange d'essai (5) est pressé à travers le tamis d'essai (10) à l'aide d'un piston (7) et d'un cylindre (6).

3. Procédé selon la revendication 1 ou 2, dans lequel un mélange d'essai (5) chauffé et d'abord mis en la forme d'un rouleau ou d'un cylindre, le mélange d'essai (5) ainsi mis en forme est introduit dans un cylindre (6) et pressé à travers le tamis d'essai (10) à l'aide d'un piston (7).

4. Procédé selon l'une quelconque des revendications précédentes, dans lequel on mélange l'additif pour caoutchouc (3) lié à un polymère et la charge d'essai (2) au moyen d'un double cylindre (1a, 1b).

5. Procédé selon l'une quelconque des revendications précédentes, dans lequel une pluralité de tamis et/ou de plaques en forme de tamis (8, 9, 10) sont disposés en étant adjacents dans le sens du pressage vu de l'arrière des tamis d'essai (10), la largeur de maille des tamis individuels (8, 9, 10) augmentant dans le sens du pressage.

6. Procédé selon l'une quelconque des revendications précédentes, dans lequel un anneau (11) est disposé avant le tamis d'essai (10).

7. Dispositif conçu pour la mise en oeuvre du procédé selon l'une quelconque des revendications précédentes, comportant un cylindre (6) et un piston (7) introduit dans celui-ci, avec une pluralité de tamis (8, 9, 10) à une extrémité du cylindre et un anneau (11) adjacent à un tamis (10).

8. Dispositif selon la revendication précédentes, dans lequel un tamis (10) présente une largeur de maille n'excédant pas 150 µm et les mailles des tamis (8, 9, 10) y faisant suite vers l'extérieur sont plus larges.

9. Dispositif selon l'une quelconque des deux revendications précédentes, comprenant un double cylindre (1a, 1b) conjointement avec des plaques de limitation (4).
